Europäisches Patentamt

European Patent Office

Office européen des brevets

(11) Veröffentlichungsnummer: **0 375 829**
**A2**

(12) **EUROPÄISCHE PATENTANMELDUNG**

(21) Anmeldenummer: **89108288.5**

(22) Anmeldetag: **09.05.89**

(51) Int. Cl.⁵: **C07C 409/24, C07C 407/00, C11D 3/39**

(30) Priorität: **06.07.88 DE 3822798**

(43) Veröffentlichungstag der Anmeldung:
**04.07.90 Patentblatt 90/27**

(84) Benannte Vertragsstaaten:
**AT BE CH DE ES FR GB IT LI LU NL SE**

(71) Anmelder: **HÜLS AKTIENGESELLSCHAFT**
**Patentabteilung / PB 15 - Postfach 13 20**
**D-4370 Marl 1(DE)**

(72) Erfinder: **Zimmermann, Frank, Dr.**
**Mittelstrasse 37**
**D-4352 Herten(DE)**
Erfinder: **Jostmann, Thomas, Dr.**
**Münsterstrasse 390**
**D-4358 Haltern 5(DE)**
Erfinder: **Schüller, Hans-Peter**
**Alte Brüderstrasse 16**
**D-4370 Marl(DE)**
Erfinder: **Engel, Klaus, Dr.**
**Löhrgasse 6**
**D-4350 Recklinghausen(DE)**

(54) **Verfahren zur Herstellung von phlegmatisierten aliphatischen Diperoxidicarbonsäuren.**

(57) Die bisherigen Verfahren zur Gewinnung von phlegmatisierten aliphatischer Diperoxidicarbonsäuren mit prozeßeigenem Natriumsulfat sind wegen der Natriumsulfatherstellung in Anwesenheit der Diperoxidicarbonsäure sicherheitstechnisch schwer und nur unter Verlusten beherrschbar; außerdem müssen Verunreinigungen wie Schwermetalle in aufwendiger Weise über die bei der Phlegmatisierung anfallenden natriumsulfathaltigen Ablaugen praktisch vollständig entfernt werden, um die Stabilität der Produkte nicht zu beeinträchtigen.

Das neue Verfahren soll die genannten Nachteile nicht aufweisen.

Es wird hierzu ein neues Verfahren vorgeschlagen, daß auf Basis von Prozeß-Natriumsulfat verbesserte Diperoxidicarbonsäuregranulate liefert, die die vorstehend genannten Nachteile nicht aufweisen.

Auf Basis von Prozeß-Natriumsulfat werden in einem sicheren Verfahren verbesserte Diperoxidicarbonsäuren gewonnen, die eine gute chemische Stabilität trotz eines hohen Anteils an Verunreinigungen, sichere Handhabbarkeit, niedrige Schüttgewichte und hohe Abriebfestigkeit aufweisen.

Herstellung von phlegmatisierten aliphatischen Diperoxidicarbonsäuren.

EP 0 375 829 A2

EP 0 375 829 A2

## Verfahren zur Herstellung von phlegmatisierten aliphatischen Diperoxidicarbonsäuren

Persauerstoffverbindungen werden in großem Umfang zur Textilbleiche eingesetzt, da sie sich außer durch gute Bleichwirkung auch durch geringe Aggressivität gegenüber Geweben auszeichnen.

Große Bedeutung bei hohen Waschtemperaturen hat Natriumperborat erlangt, daß als sicher handhabbares, mildes Oxidationsmittel gute Bleichergebnisse liefert. Der Nachteil dieses Bleichmittels liegt in der nicht zufriedenstellenden Bleichwirkung bei niedrigen Waschtemperaturen. Das steigende Aufkommen von temperaturempfindlichen Synthesefasern und farbigen Textilien sowie der Trend zum Energieeinsparen beim Waschprozeß führt unter anderem zu zunehmender Bedeutung der Niedrigtemperaturbleiche und macht somit die Suche nach aktiveren Oxidationsmitteln notwendig.

Als geeignete Niedrigtemperaturbleichmittel erwiesen sich Peroxicarbonsäuren. Ihr Nachteil ist die große Neigung zu exothermer Zersetzung bzw. Explosion in reiner Form bei thermischer bzw. mechanischer Beanspruchung, so daß eine gefahrlose Handhabung ohne geeignete Schutzmaßnahmen nicht möglich ist.

Es wurden Verfahren zur gefahrlosen Handhabung entwickelt, wie etwa die Erzeugung der bleichaktiven Persauerstoffverbindung erst in der Waschflotte durch Reaktion ungefährlicher Persauerstoffverbindungen (z. B. Natriumperborat) mit Aktivatoren, jedoch sind diese Verfahren mit dem Nachteil behaftet, daß hohe Konzentrationen an Ausgangspersauerstoffverbindung und Aktivator vorhanden sein müssen, um die Verluste durch die Hydrolyseempfindlichkeit der Aktivatoren, die Anfälligkeit auf Schwankungen der Dosierung und die unterschiedliche Lösungsgeschwindigkeit der Komponenten an bleichaktiver Spezies auszugleichen (vgl. hierzu A. Smith et. al., Low Temperature Bleach Systems, AOCS World Conference on Detergents, Montreaux 1986).

Darüberhinaus kann sich durch die temperaturabhängige Geschwindigkeit der Aktivierungsreaktion insbesondere bei niedrigen Temperaturen die Erzeugung ausreichender Konzentrationen an bleichaktiver Substanz unerwünscht lange verzögern.

Da diese Schwierigkeiten beim direkten Einsatz von Peroxicarbonsäuren nicht auftreten können, ist man seit langem bemüht, die thermische und mechanische Sensibilität von Peroxicarbonsäuren durch Phlegmatisierung zu reduzieren.

Unter den verfügbaren Peroxicarbonsäuren konzentriert sich das Interesse auf längerkettige aliphatische Peroxicarbonsäuren, die einen Aktivsauerstoffgehalt von weniger als 14 % aufweisen. Besonders günstige anwendungstechnische Eigenschaften besitzen lineare alpha,omega-Diperoxidcarbonsäuren, da sie nahezu keinen Eigengeruch und gute Bleichwirkung selbst bei Temperaturen von 30 bis 40 $^\circ$C aufweisen. Darüberhinaus verfügen sie über tensidische Eigenschaften, wodurch sich die Bleichwirkung nicht wie beim Perborat statistisch überall in der Waschflotte entfaltet, sondern relativ spezifisch direkt am Gewebe. Eine von Lieser et al. (Seifen, Öle, Fette, Wachse 111, 452 (1985) publizierte systematische Untersuchung der Bleichwirkung in Abhängigkeit von der Kettenlänge zeigt ein Maximum bei C = 12, d. h. für die Diperoxidodecandisäure. Da der für die Herstellung von Diperoxidodecandisäure benötigte Rohstoff Dodecandisäure zudem noch großtechnisch verfügbar ist, kommt der Diperoxidodecandisäure eine herausragende Bedeutung zu.

Für die Herstellung von wasserlöslichen alpha,omega-Diperoxidicarbonsäuren existieren verschiedene Verfahren, deren Grundprinzip nach der DE-AS 10 48 569 darin besteht, daß die entsprechenden alpha,omega-Dicarbonsäuren in wäßrigem Medium bei Temperaturen ab -50 $^\circ$C mit Wasserstoffperoxid unter saurer Katalyse, meist Schwefelsäure, umgesetzt werden. Nach gleichem Reaktionsprinzip arbeiten die Verfahren der US-PSS 4 119 660, 4 244 884, 4 314 949, DE-OSS 28 61 690, 32 01 579, 33 20 497 und 34 18 450 die teilweise auch kontinuierliche Reaktionsführung zulassen. Als Reaktionsaustrag erhält man meist Suspensionen mit Feststoffgehalten von 2 bis 36 %, deren flüssige Phase aus 60 bis 80 % Schwefelsäure neben Resten an Wasserstoffperoxid besteht.

Die Weiterverarbeitung der Reaktionsausträge wird ebenfalls in der US-PS 4 119 660 und DE-OS 28 61 690 beansprucht; es wird dort die unlösliche Diperoxidicarbonsäure durch Filtration abgetrennt, gefolgt von einer Trocknung.

Diese Verfahrens sind mit dem entscheidenden Nachteil behaftet, daß hierbei hochkonzentrierte Diperoxidicarbonsäuren auftreten, die äußerst sensibel gegen mechanische und thermische Beanspruchung sind. Es läßt sich somit keine sichere technische Durchführung gewährleisten.

Demgegenüber wird in den DE-OSS 33 20 496, 33 20 497 und 34 18 450 der saure Reaktionsaustrag direkt mit Alkalihydroxiden neutralisiert, so daß dort ein Gemisch aus Alkalisufalt und Diperoxidicarbonsäure abgetrennt wird. Nachteilig ist jedoch, daß durch die bei der Neutralisation auftretenden Temperatur- und pH-Spitzen im alkalischen Bereich eine rasche Zersetzung der Peroxicarbonsäuren bewirkt wird und somit

2

hohe Verluste an dem Produkt auftreten.

Somit steht bislang noch kein Verfahren zur Verfügung, das es gestattet, bei der Peroxigenierung anfallende Diperoxidicarbonsäuresuspensionen in sicherer Weise ohne signifikante Verluste an Aktivsubstanz weiterzuverarbeiten.

Da Peroxicarbonsäuren als solche in reiner Form nicht sicher handhabar sind, müssen diese Bleichmittel phlegmatisiert werden.

So ist seit langem bekannt, daß Peroxicarbonsäuren entsprechend der BE-PS 560 389 durch Mischen mit hydratisierbaren anorganischen Salzen, insbesondere Natriumsulfat und Magnesiumsulfat in eine ausreichend phlegmatisierte, sicher handhabbare Form überführt werden können. Zur Durchführung des Mischprozesses wurden zahlreiche Verfahren entwickelt. So schlägt die DE-OS 20 38 833 vor, die Phlegmatisierung von wäßrigen, gefrorenen Peroxicarbonsäuretröpfchen in einem Fließbett aus Magnesiumsulfat durchzuführen, so daß die Peroxicarbonsäure vom hydratisierten Magnesiumsulfat umhüllt wird. Ein ähnliches Verfahren empfiehlt die DE-AS 16 68 569, wobei eine wäßrige Peroxicarbonsäuresuspension in das Wirbelbett eines Salzhydrates eingebracht wird.

Demgegenüber beschreiben die DE-OS 24 22 735 Verfahren, in denen die trockene oder nahezu trockene Peroxicarbonsäure mechanisch mit dem als Phlegmatisierungsmittel gewählten Salz vermischt wird.

Ein weiterer Problembereich bei diesen Peroxicarbonsäuren ist ihre schlechte chemische Stabilität bei der Lagerung. Als Ursache für die schlechte chemische Stabilität wurde die Anwesenheit von Schwermetallionen postuliert, die als äußerst wirksame Zersetzungskatalysatoren für Peroxide bekannt sind (siehe R. Criegee in Houben-Weil, Band 8, 1952).

Zur Verbesserung der chemischen Stabilität schlagen die DE-ASS 11 74 755, 12 80 239 und DE-OSS 29 38 731 und 22 14 500 vor, Komplexierungsmittel für Schwermetalle, wie Chinaldinsäure, Chinolin, Polyphosphate, Aminophosphonsäuren und EDTA als Additive zuzusetzen. Durch diese Maßnahmen erhält man Endprodukte mit verbesserter, aber immer noch nicht zufriedenstellender chemischer Stabilität.

Um die Phlegmatisierung von Peroxicarbonsäuren mit Natriumsulfat wirtschaftlich tragbar zu gestalten und um Entsorgungsprobleme von Schwefelsäure oder Natriumsulfatlösung weitgehend zu vermeiden, ist es sinnvoll, daß für die Phlegmatisierung benötigte Natriumsulfat im wesentlichen aus der anfallenden Prozeßschwefelsäure zu erzeugen. Daher schlägt die DE-OS 36 28 263 vor, z. B. gemäß der DE-OS 33 20 497 hergestellte Reaktionsmischungen mit einem gereinigten, aus einer recyclierten Ablauge gewonnenen, vorzugsweise als gesättigte wäßrige Lösung vorliegenden Natriumsulfat zu versetzen, die so entstandene Mischung mit Natronlauge zu neutralisieren und anschließend die mit Natriumsulfat phlegmatisierte Peroxicarbonsäure von der Mutterlauge abzutrennen.

Aus der an Natriumsulfat gesättigten Mutterlauge wird durch Kristallisation reines Natriumsulfat gewonnen, das bevorzugt als wäßrige Lösung in die anfallende Reaktionsmischung recycliert wird. Bei diesem Verfahren ist es unumgänglich, praktisch alle im Phlegmatisierungsprozeß vorhandenen Verunreinigungen zu entfernen, um die Lagerstabilität der phlegmatisierten Peroxicarbonsäuren nicht zu verschlechtern. Dazu müssen vor allem die bei dieser Verfahrensweise besonders kritischen Schwermetallspuren über die natriumsulfathaltigen Mutterlaugen weitgehend ausgeschleust werden, wobei die Schwermetallkonzentration im recyclierten Natriumsulfat höchstens 5 ppm und in den Endprodukten im allgemeinen höchstens 2 ppm betragen darf. Dieses Verfahren verringert zwar die Mengen an natriumsulfathaltigen Ablaugen, jedoch werden phlegmatisierte Peroxicarbonsäuren erhalten, die trotz eines extrem niedrigen Gehaltes an Verunreinigungen, insbesondere Schwermetallen, keine verbesserte Lagerstabilität besitzen. Darüberhinaus ist es zum einen verfahrenstechnisch aufwendig, Verunreinigungen im ppm-Bereich nahezu vollständig abzutrennen, zum anderen besteht auch ein Nachteil in der Gefahr, daß bei Schwankungen der Qualität der Einsatzmaterialien oder bei Prozeßstörungen sehr rasch ein nicht tolerierbarer Verunreinigungspegel erreicht wird, der zu Nachteilen hinsichtlich Lagerstabilität und sicherer Handhabbarkeit führt.

Außer den Problemen der sicheren Handhabung und chemischen Stabilität unterliegen Peroxicarbonsäureformulierungen jedoch noch verschiedenen anderen Anforderungen. Da das Hauptanwendungsgebiet von Peroxicarbonsäuren die Textilbleiche ist, müssen Peroxicarbonsäureformulierungen für die Einarbeitung in Waschmittel eine den üblichen Waschmittelbestandteilen entsprechende Spezifikation aufweisen. Zur Gewährleistung einer guten Rieselfähigkeit und zur Vermeidung von Entmischungsvorgängen im Waschmittel ist es vorteilhaft, feste Peroxicarbonsäureformulierungen auf einen Korngrößenbereich von 200 bis 1 250 μm und auf Schüttgewichte von 400 bis 800 g/l einzustellen. Dieses Anforderungsprofil kann prinzipiell durch Aufbaugranulierung erreicht werden.

So beschreiben die DE-OS 35 15 712 und die EP-A 0 256 443 Verfahren zur Aufbaugranulierung, bei denen feste, phlegmatisierte Peroxicarbonsäuren unter Verwendung eines wasserlöslichen Polymeren als Granulierhilfsmittel agglomeriert werden. Die Aufbaugranulierung wird hierbei erreicht durch Aufsprühen einer wäßrigen Lösung des Granulierhilfs mittels auf die bewegte, phlegmatisierte Peroxicarbonsäure, nach der DE-OS 35 15 712 auch durch trockenes Vermischen des Granulierhilfsmittels mit der phlegmatisierten Peroxicarbonsäure und anschließendem Aufsprühen von Wasser. Ein ähnliches Verfahren beansprucht die GB-PS 8 127 157, wobei Polyvinylalkohole und -Derivate bevorzugt zur Granulierung im Fießbett verwendet werden.

Durch diese Maßnahmen lassen sich spezifikationsgerechte Aufbaugranulate herstellen. Nach H. Telschig (Seifen, Öle, Fette, Wachse 1984, 1) ist aber bekannt, daß die Schüttdichte der Endprodukte im wesentlichen durch die Schüttgewichte der Ausgangsprodukte bestimmt werden. So erreicht man zwar durch Aufbaugranulierung im Normalfall eine Schüttgewichtsreduzierung um 5 bis 10 %, in Ausnahmefällen um bis zu 17 %, jedoch sind Granulate mit niedrigen Schüttgewichten nur dann herstellbar, wenn Vorprodukte mit entsprechend niedrigem Schüttgewicht zur Verfügung stehen.

Obwohl durch die beschriebenen Maßnahmen eine Reihe von Problemen gelöst werden, stehen bislang noch keine ausreichend lagerstabilen und sicher handhabbaren Peroxicarbonsäureformulierungen zur Verfügung, die gefahrlos hergestellt werden können und eine den üblichen Waschmittelbestandteilen entsprechende Spezifikation aufweisen. Darüberhinaus ist auch der Einsatz von Prozeß Schwefelsäure bzw. daraus hergestelltem Natriumsulfat zur Phlegmatisierung noch nicht zufriedenstellend gelöst.

Dieser Erfindung liegt daher die Aufgabe zugrunde, mittels eines sicherheitstechnisch unbedenklichen Verfahrens verbesserte Diperoxidicarbonsäuregranulate, die die Nachteile des Standes der Technik nicht aufweisen, auf Basis von Prozeß-Natriumsulfat zu entwickeln.

Erfindungsgemäß gelöst wird die Aufgabe, indem man den als Suspension anfallenden Reaktionsaustrag, im wesentlichen bestehend aus ungelöster Diperoxidicarbonsäure und wäßriger Schwefelsäure unter Vermeidung von Zonen mit nahezu trockener Diperoxidicarbonsäure durch Filtration auftrennt in ein Filtrat aus wäßriger Schwefelsäure und eine Diperoxidicarbonsäure und wenig Schwefelsäure enthaltende wäßrige Suspension, wonach man beide Fraktionen mit Natronlauge neutralisiert. Aus der praktisch diperoxidicarbonsäurefreien Natriumsulfatlösung stellt man durch zweistufige Kristallisation ein gereinigtes wasserfreies Natriumsulfat her. In der diperoxidicarbonsäurehaltigen Suspension werden wasserlösliche Polymere und ggf. zusätzliche Additive gelöst, wonach man die Suspension durch Vermischen mit dem hergestellten Natriumsulfat dehydratisiert unter Ausbildung von Aufbaugranulaten. Diese Aufbaugranulate sind gekennzeichnet, durch gute chemische Stabilität, sichere Handhabbarkeit, niedrige Schüttgewichte und hohe Abriebfestigkeit. Darüberhinaus ermöglichen die angewandten Verfahren eine gefahrlose Herstellung der Granulate auf Basis der anfallenden Reaktionsausträge.

Überraschenderweise hat sich gezeigt, daß im erfindungsgemäßen Verfahren eine ausgeprägte Toleranz gegenüber Schwermetallen besteht, so daß sich selbst aus Reaktionsmischungen mit bis zu 40 ppm Schwermetallen, die bei Verwendung technischer Materialien auftreten, gute Produkte herstellen lassen.

Gegenstand der Erfindung ist ein Verfahren, welches dadurch gekennzeichnet ist, daß man

a) den Reaktionsaustrag der mittels Wasserstoffperoxid/Schwefelsäure durchgeführten Peroxigenierung von wasserunlöslichen Dicarbonsäuren, mit oder ohne Verdünnen mit Wasser, durch kontinuierliche Filtration in einer mehrstufigen Filtrationskaskade, die das Auftreten von Zonen mit nahezu trockener Diperoxidicarbonsäure ausschließt, auftrennt in ein die Hauptmenge an Schwefelsäure enthaltendes, weitgehend peroxidfreies Filtrat und eines bezogen auf die flüssige Phase der Suspension weniger als 10 % Schwefelsäure enthaltende Diperoxidicarbonsäuresuspension,

b) aus der als Filtrat erhaltenen Schwefelsäure in an sich bekannter Weise nach Neutralisation auf pH 2 bis 6 mit bis zu 50%iger wäßriger Natronlauge, die bis zu 30 ppm Schwermetalle und weniger als 50 ppm Chloridionen enthält, eine Natriumsulfatlösung herstellt, aus der, mit oder ohne Verdünnen mit Wasser oder Aufkonzentrieren, durch Kühlungskristallisation zunächst Glaubersalz und anschließend durch Verdampfungskristallisation ein Natriumsulfat mit einem Schüttgewicht von 1 000 bis 1 500 g/l und einer Korngrößenverteilung von mindestens 90 % unterhalb 200 μm, gewonnen wird, das bis zu 30 ppm Schwermetalle, bis zu 50 ppm Chloridionen und weniger als 5 % Wasser enthält,

c) die Schwefelsäure der nach Filtration anfallenden Diperoxidicarbonsäuresuspension bei Temperaturen unterhalb von 40 °C, durch Zugabe bis zu 50%iger wäßriger Natronlauge, die bis zu 30 ppm Schwermetalle und bis zu 50 ppm Chloridionen enthält, auf pH 2 bis 6 neutralisiert,

d) der nach Neutralisation erhaltenen Diperoxidicarbonsäuresuspension, bezogen auf den Gehalt an Diperoxidicarbonsäure, 2 bis 200 % eines wasserlöslichen Polymeren zusetzt, wobei die Schwermetallkonzentration in der Diperoxidicarbonsäuresuspension nach Zugabe des Polymeren bis zu 30 ppm und die Chloridionenkonzentration bis zu 50 ppm beträgt,

e) und die Diperoxidicarbonsäure mit einer Schicht, bestehend aus dem unter d) zugesetzten Polymeren umgibt, wobei man die gemäß d) erzeugte wäßrige Diperoxidicarbonsäuresuspension durch Vermischen mit dem unter b) hergestellten Natriumsulfat in geeigneten Apparaturen dehydratisiert, so daß

4

gleichzeitig Aufbaugranulate entstehen, die nach dem schonenden Trocknen zu mindestens 90 % Korngrößen im Bereich von 200 bis 1 250 μm und Schüttgewichte zwischen 400 bis 800 g/l aufweisen und die bei zweistündiger Messung der Abriebfestigkeit auf einen Engelsmann-Taumelsieb der Maschenweite 200 μm weniger als 6 % Abrieb liefern.

Die nach dem erfindungsgemäßen Verfahren, das eine gefahrlose Herstellung der Granulate auf Basis der anfallenden Reaktionsausträge ermöglicht, hergestellten Aufbaugranulate sind gekennzeichnet durch gute chemische Stabilität trotz eines hohen Anteils an Verunreinigungen, sowie durch sichere Handhabbarkeit, niedrige Schüttgewichte und hohe Abriebfestigkeit.

Als organische Diperoxidicarbonsäuren können alle Diperoxidicarbonsäuren verwendet werden, die sich durch sauerkatalysierte Reaktion der entsprechenden Dicarbonsäuren mit Wasserstoffperoxid herstellen lassen und die keine nennenswerte Wasserlöslichkeit aufweisen. Nicht nennenswert wasserlöslich sind solche Diperoxidicarbonsäuren, deren Löslichkeit in Wasser bei Raumtemperatur weniger als 1 % beträgt. Bevorzugte Diperoxidicarbonsäuren sind alpha,omega-Diperoxidicarbonsäuren mit 9 bis 13 Kohlenstoffatomen und insbesondere Diperoxidodecandisäure sowie Diperoxibrassylsäure und Gemische.

Als Ausgangsmaterial für die Herstellung phlegmatisierter Diperoxidicarbonsäure-Granulate dient der bei der Peroxigenierung anfallende Reaktionsaustrag. Üblicherweise führt man derartige Peroxigenierungen kontinuierlich in einer mehrstufigen, vorzugsweise 3stufigen Rührkesselkaskade durch, indem man die wasserunlösliche Ausgangsdicarbonsäure vorzugsweise in fester Form mit einer Mischung aus Schwefelsäure und Wasserstoffperoxid umsetzt. Wirtschaftlich tragbare Verfahren können jedoch nur bei Verwendung großtechnisch verfügbarer Ausgangsmaterialien realisiert werden. Hierbei ist zu beachten, daß technische Substanzen Schwermetalle, vor allem Eisen, im ppm-Bereich enthalten. Ionen von Schwermetallen wie Nickel, Chrom, Cobalt, Zink, Mangan, Cadmium und Blei, vor allem aber Eisen und Kupfer sind ausgeprägte Zersetzungskatalysatoren für Peroxide.

.Die flüssige Phase der Reaktionsmischung besteht zu Beginn aus 50 bis 70 % Schwefelsäure, 4 bis 20 % Wasserstoffperoxid und 10 bis 46 % Wasser, die feste Phase aus 1 bis 25 % Dicarbonsäure, die bevorzugt als Pulver mit einer mittleren Korngröße von weniger als 100 μm zudosiert wird. Die Reaktion wird bei Temperaturen von 30 bis 50 °C durchgeführt. Bei Verweilzeiten von 4 bis 14 Stunden erreicht man so Umsätze von mehr als 90 %, in der Regel mehr als 95 %. Unter diesen Bedingungen kann ein oxidativer Abbau der Dicarbonsäure/Diperoxidicarbonsäure weitgehend vermieden werden, so daß praktisch keine Verluste auftreten. Die in der Reaktionsmischung gelösten Verunreinigungen aus diesem oxidtiven Abbau der Säuren liegen unter 1 %.

Als Reaktionsaustrag erhält man nach beendeter Reaktion eine Suspension, deren feste Bestandteile aus 1 bis 25 % der erzeugten Diperoxidicarbonsäure und Resten nicht umgesetzter Ausgangsdicarbonsäuren besteht, während als flüssige Phase eine 50 bis 70%ige Schwefelsäure erhalten wird, die außer gelösten organischen Verunreinigungen noch Reste an überschüssigem Wasserstoffperoxid enthalten kann. Das Verhältnis Diperoxidicarbonsäure beträgt mindestens 90 : 10, in der Regel mindestens 95 : 5.

Der Reaktionsaustrag wird durch Filtration aufgetrennt in ein, die Hauptmenge an Schwefelsäure enthaltendes Filtrat und eine Diperoxidicarbonsäuresuspension in verdünnter Schwefelsäure. Dadurch wird ermöglicht, aus der als Filtrat anfallenden Schwefelsäure ohne die Anwesenheit der Diperoxidicarbonsäure nach der Neutralisation mit Natronlauge ein kristallines Natriumsulfat herzustellen. Die separate Weiterverarbeitung der Schwefelsäure ist besonders vorteilhaft, da bei Abwesenheit der Diperoxidicarbonsäure keine besonderen sicherheitstechnischen Maßnahmen notwendig sind.

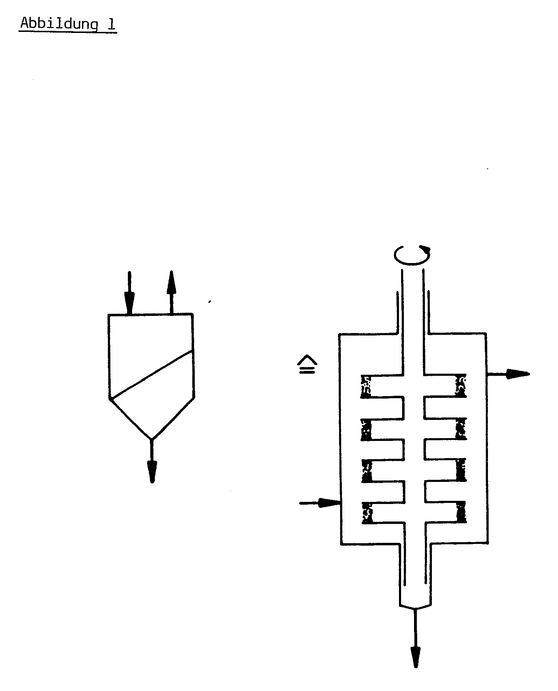

Aus Sicherheitsgründen ist es erforderlich, daß durch die Filtration eine Diperoxidicarbonsäuresuspension mit Feststoffgehalten von 10 bis 40 %, vorzugsweise 20 bis 35 %, erzeugt wird, deren flüssige Phase weniger als 10 %, bevorzugt weniger als 5 %, Schwefelsäure enthält. Zur gefahrlosen Durchführung muß zudem sichergestellt sein, daß bei der Filtration keine Zonen mit nahezu trockener Diperoxidicarbonsäure auftreten. Prinzipiell geeignet sind hierfür alle Filtrationsverfahren, bei denen kein significanter Filterkuchen aufgebaut wird. Als erfindungsgemäß geeignete Verfahren erweisen sich vor allem die Querstrommikrofiltration sowie die Filtration mit bewegten Filterkerzen geeigneter Größe. Besonders bevorzugt ist die Filtration durch radial angeordnete Filterkerzen (Abb. 1), wobei durch deren Rotation auch eine ausreichende Rührung der Suspension erreicht und der Aufbau eines Filterkuchens weitgehend vermieden wird. Sofern die zu filtrierende Diperoxidicarbonsäuresuspension gepumpt werden muß, ist es zur Vermeidung von Sicherheitsrisiken notwendig, Pumpen zu verwenden, in denen möglichst geringe Scherkräfte auftreten.

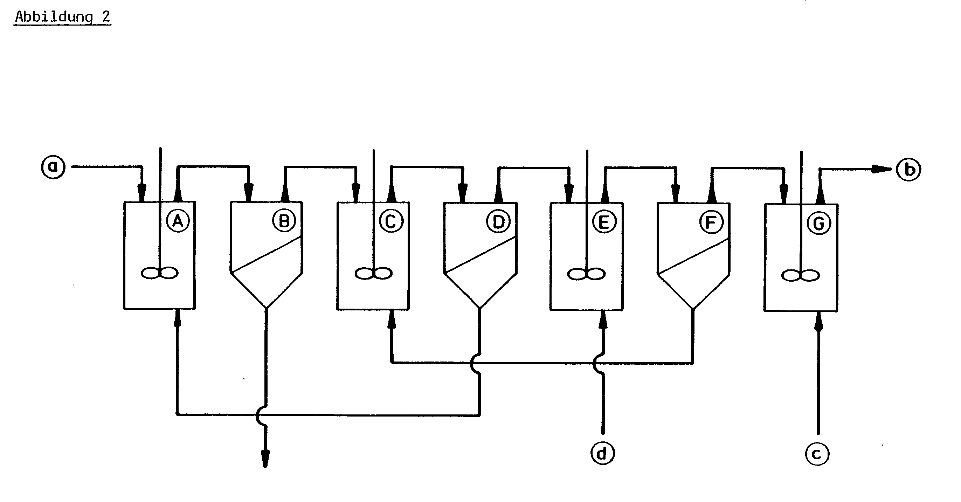

Die erfindungsgemäß favorisierte Filtration besteht aus einer mehrstufigen, bevorzugt 2 bis 4stufigen, insbesondere 3stufigen kontinuierlichen Filtration nach dem Prinzip der Querstrommikrofiltration oder mit bewegten Filterkerzen, wie in Abb. 2 am Beispiel einer 3stufigen Filtration dargestellt. In dieser Abbildung bedeuten a = Zustrom Reaktionsmischung, b = Abstrom konfektionierte Diperoxidicarbonsäuresuspension, c = Zustrom Additive und Neutralisation, d = Zustrom Wasser, A, C und E = Mischer, B, D und F = Filtrationseinheiten und G = Neutralisation und Konfektionierung. In den Rührkessel A werden der Reaktionsaustrag und in den Rührkessel E 0,1 bis 5,5, vorzugsweise 0,5 bis 2,5, Teile Wasser pro Teil Reaktionsaustrag kontinuierlich eingespeist. Das in den Filtrationseinheiten F und D anfallende Filtrat wird vorzugsweise vollständig in die jeweils vorhergehende Filtrationsstufe zurückgeführt. So wird in Abb. 2 das Filtrat aus D in den Rührkessel A zurückgeführt. Durch diese Maßnahme erhält man nach Erreichen des stationären Zustands eine 10 bis 40%ige, bevorzugt 25 bis 35%ige, Schwefelsäure als Filtrat sowie eine, bezogen auf die flüssigen Anteile, weniger als 10 %, bevorzugt weniger als 5 %, Schwefelsäure enthaltende Suspension mit einem auf die Gesamtmenge der Suspension bezogenen Gehalt an Diperoxidicarbonsäure von 10 bis 40 %, vorzugsweise 20 bis 35 %.

Zweckmäßigerweise wird die Diperoxidicarbonsäuresuspension anschließend in einen nachgeschalteten Rührkessel überführt, wo sie bei Temperaturen unterhalb 40 °C, vorzugsweise unterhalb 20 °C, durch Zugabe bis zu 50%iger, vorzugsweise 30 bis 50%iger, wäßriger Natronlauge auf pH 2 bis 6, bevorzugt 3 bis 4, neutralisiert wird. Hierbei zeigt sich, daß mit der Neutralisation keine signifikanten Verluste an Diperoxidicarbonsäure mehr verbunden sind, wenn die Schwefelsäurekonzentration weniger als 10 % beträgt und die Neturalisationstemperatur unter 40 °C liegt.

Entscheidendes Kriterium für die Filtration ist daher die Erzeugung einer Diperoxidicarbonsäuresuspension mit, bezogen auf die flüssigen Anteile, weniger als 10 %, bevorzugt weniger als 5 %, Schwefelsäure. Dies kann prinzipiell zwar auch in einer einstufigen Fahrweise realisiert werden, jedoch muß dann der Reaktionsaustrag unzweckmäßig stark verdünnt werden. Je niedriger die gewählte Stufenzahl der filtration ist, umso mehr Wasser wird zur Verdünnung benötigt, damit die erforderliche Reduktion der Schwefelsäurekonzentration in der Suspension erreicht wird. Für die Weiterverarbeitung der als Filtrat anfallenden Schwefelsäure sollte die Schwefelsäurekonzentration zwischen 10 bis 40 %, bevorzugt zwischen 25 bis 35 %, liegen. Um dies zu erreichen, ist eine 2 bis 4stufige Filtration bevorzugt, insbesondere eine 3stufige. Der in den jeweiligen Filtrationsstufen einzustellende Gehalt an Diperoxidicarbonsäure soll zwischen 10 bis 40 %, bevorzugt 25 bis 30%, liegen wobei der Feststoffgehalt in allen Stufen auf das gleich oder auf unterschiedliche Niveaus eingestellt werden kann. Niedrigere Feststoffgehalte als 10 % sind in Hinblick auf die Weiterverarbeitung unzweckmäßig, bei höheren Feststoffgehalten als 40 % ist die Suspension in der Regel nicht mehr ausreichend rühr- und pumpfähig.

Die Neutralisation der in der Diperoxidicarbonsäuresuspension verbleibenden Schwefelsäure wird mit Natronlauge durchgeführt. Wegen der direkten Weiterverarbeitung dieser Suspension muß die verwendete Natronlauge gewissen Reinheitskriterien genügen. So sollte der Chloridionengehalt weniger als 50 ppm betragen. Die Schwermetallkonzentration ist nicht kritisch und kann bis zu 30 ppm betragen.

So erzeugte Diperoxidicarbonsäuresuspension können entweder direkt weiterverarbeitet oder vorzugsweise zunächt mit Additiven versetzt werden. Aufgabe der zugesetzten Additive ist es, die chemische Stabilität der Diperoxidicarbonsäure oder die mechanische Stabilität der granulierten Endprodukte zu verbessern. Als erfindungsgemäß besonders günstig erwies sich der Einsatz von, bezogen auf den Gehalt an Diperoxidicarbonsäure, 0 bis 20 %, vorzugsweise 2 bis 20 %, eines Phosphates, bevorzugt Natriumtripolyphosphat, 0 bis 150 %, bevorzugt 20 bis 100 %, Magnesiumsulfat-Heptahydrat, 0 bis 10 %, bevorzugt 0 bis 3 %, eines Komplexierungsmittels für Schwermetalle, 0 bis 20 %, bevorzugt 0 bis 5 % eines Silikates, bevorzugt Natriummetasilikat sowie 2 bis 200 %, bevorzugt 5 bis 50 %, eines wasserlöslichen organischen Polymeren. Als wasserlösliche Polymere sind insbesondere polymere Carbonsäuren geeignet, vorzugsweise Homopolymerisate ungesättigter Carbonsäuren, wie z. B. Acrylsäure, Maleinsäure und Crotonsäure sowie deren Copolymerisate untereinander und mit Ethylen, Propylen, Butenen, Vinylacetat und Styrol. Neben dem Zusatz von Magnesium sulfat-Heptahydrat ist es alternativ auch möglich, Magnesiumsulfat in situ zu erzeugen, indem man die in der Diperoxidicarbonsäuresuspension nach Filtration noch enthaltene Schwefelsäure vollständig oder teilweise mit Magnesiumoxid oder Magnesiumhydroxid vornimmt. Zur Komplexierung von Schwermetallen sind im Bedarfsfall die üblichen Komplexierungsmittel geeignet, bevorzugt vor allem Chinaldinsäure, Chinolin, Aminophosphorsäuren, Ethylendiamintetraacetat und Diethylentriaminpentaacetat.

Auch beim Zusatz der Additive erfolgt eine mögliche Kontaminierung mit Chloridionen, so daß darauf zu achten ist, daß nach Zusatz der Additive die Chloridkonzentration unter 50 ppm bleibt. Der Schwermetallgehalt ist nicht kritisch und kann nach Zusatz der Additive bis zu 30 ppm betragen.

Nach der Filtration fällt als Filtrat eine 10 bis 40%ige, bevorzugt 25 bis 35%ige, Schwefelsäure an, die noch Reste an überschüssigem Wasserstoffperoxid enthalten kann.

Es hat sich gezeigt, daß es nach der erfindungsgemäßen, im wesentlichen vollständigen Seperation von Diperoxidicarbonsäure und Schwefelsäure ausreicht, in an sich bekannter Weise die Schwefelsäure durch Neutralisation und Kristallisation in Natriumsulfat umzuarbeiten. Diese technisch einfache Weiterverarbeitung wird ermöglicht durch die überraschend hohe Toleranz des erfindungsgemäßen Verfahrens gegenüber Schwermetallspuren in Natriumsulfat. Die anfallende Schwefelsäure wird bei Temperaturen von 30 bis 60 °C, vorzugsweise 35 bis 45 °C, mit bis zu 50%iger Natronlauge, die weniger als 50 ppm Chloridionen, sowie weniger als 30 ppm Schwermetalle enthält, auf pH 2 bis 6 neutralisiert, wonach man abkühlt und das ausgefallene Glaubersalz durch Zentrifugieren oder Filtrieren abtrennt. Das Glaubersalz wird bei Temperaturen von 32 bis 70 °C aufgeschmolzen und einer Verdampfungskristallisation unterzogen. Bei Temperaturen von 60 bis 110 °C und Drucken von 10 bis 200 mbar setzt man die Verdampfungskristallisation so lange fort, bis ein Feststoffgehalt von 15 bis 50 % erreicht wird, worauf man das Natriumsulfat durch Filtrieren oder Zentrifugieren abtrennt und z. B. in einem Konvektions- oder Kontakttrockner bis auf Restfeuchten unterhalb 0,5 % trocknet. Dadurch erhält man ein Natriumsulfat mit einem Schüttgewicht von 1 000 bis 1 500 g/l und einer Korngrößenverteilung von mindestens 90 % unterhalb 200 μm, bevorzugt unterhalb 100 μm. Nach dieser Kristallisation liegt der Chloridgehalt unter 50 ppm.

Der Schwermetallgehalt kann bei Weiterverarbeitung nach dem erfindungsgemäßen Verfahren ohne signifikante Beeinträchtigung der Stabilität des Endproduktes bis zu 30 ppm betragen.

Das granulierte Endprodukt wird hergestellt, indem man das prozeßeigene kristalline Natriumsulfat mit einer Diperoxidicarbonsäuresuspension vermischt. Im Mischprozeß erfolgt eine Dehydratisierung der Diperoxidicarbonsäure enthaltenden Suspension, wobei das mit der Suspension eingebrachte Wasser als Kristallwasser im Natriumsulfat gebunden wird. Trotz des hohen Schwermetallgehaltes führt die Verwendung des prozeßeigenen Natriumsulfates zu einer drastischen Erhöhung von chemischer Stabilität sowie Erniedrigung der Zersetzungsneigung.

Erfindungsgemäß besonders vorteilhaft ist jedoch die Verwendung von Diperoxidicarbonsäuresuspensionen unter Zusatz der stabilisierenden Additive, die dann im Endprodukte in räumlicher Nachbarschaft zur Diperoxidicarbonsäure angereichert werden, wodurch nur eine relativ niedrige Konzentration an Additiven erforderlich ist. Entscheidende Bedeutung für die weitere Verbesserung der chemischen und mechanischen Stabilität der Granulate kommt dem wasserlöslichen Polymeren zu.

Die durch das Mischen der Suspension mit Natriumsulfat bewirkte Dehydratisierung führt zu einer Umhüllung der Diperoxidicarbonsäure mit dem Polymeren. Die damit verbundene effektive Abschirmung der Diperoxidicarbonsäure von zersetzungsfördernden Bestandteilen macht sich in einer deutlichen Verbesserung der chemischen Stabilität bemerkbar, was gleichzeitig die Toleranz gegenüber Schwermetallspuren im Natriumsulfat verbessert.

Darüberhinaus wird durch das gelöste Polymere ein sehr effektives Verkleben der einzelnen Bestandteile unter Bildung von Aufbaugranulaten erreicht, so daß mechanisch überraschend stabile Endprodukte mit ausgeprägtem Instantcharakter entstehen. Durch die ausgezeichneten Klebeeigenschaften der benutzten Polymeren wird auch eine drastische Reduktion des Schüttgewichtes erreicht. Erfindungsgemäß benutzt wird prozeßeigenes, gereinigtes Natriumsulfat, das nach Kristallisation und Trocknen als Schwersulfat mit einem Schüttgewicht von 1 000 bis 1 500 g/l und einer Korngrößenverteilung von mindestens 90 % unterhalb 200 μm, bevorzugt unterhalb 100 μm, anfällt. Bei Verwendung dieses Natriumsulfates erhält man nach dem Mischen mit der gelöstes Polymer enthaltenden Suspension ein Aufbaugranulat, das nach dem schonenden Trocknen Schüttgewichte von 400 bis 800 g/l, vorzugsweise 500 bis 650 g/l, und Korngrößen von mindestens 90 % im Bereich von 200 bis 1 250 μm aufweist. Die ausgezeichnete mechanische Stabilität der Aufbaugranulate wird bei Messung der Abriebfestigkeit auf einem Engelsmann-Taumelsieb der Maschenweite 200 μm ersichtlich. Zweistündige mechanische Belastung liefert hierbei wenigier als 6 % Abrieb in Form von Partikeln mit Korngrößen unterhalb 200 μm.

Werden außer dem wasserlöslichen Polymeren noch andere Additive der Suspension zugesetzt, so kann eine weitere Verbesserung der chemischen Stabilität erreicht werden. Insbesondere bei Kombinationen von wasserlöslichen Polymeren mit Magnesiumsulfat und Phosphaten, vorzugsweise Natriumtripolyphosphat, tritt eine synergistische Verbesserung der chemischen Stabilität auf, die bei alleiniger Verwendung der einzelnen Additive nicht zu erreichen ist. Darüberhinaus wird auch eine deutliche Erhöhung der Zersetzungstemperatur der getrockneten Granulate erzielt.

Die Durchführung des Mischverfahrens kann im kommerziell erhältlichen Agglomeratoren nach dem Sprühmischprinzip erfolgen. Um eine homogene Verteilung der Diperoxidicarbonsäure in den Granulaten zu erreichen, wird die Diperoxidicarbonsäuresuspension, vorzugsweise feinverteilt, mittels einer Düse oder Sprühwelle auf bewegtes Natriumsulfat aufgebracht.

Erfindungsgemäß besonders geeignete Geräte sind Agglomeratoren mit ro tierenden Trommeln oder Kegeln, kontinuierlich arbeitende vertikale Agglomeratoren, kontinuierliche "Zig-Zag"-Agglomeratoren sowie Wirbelschichtgranulatoren. Bevorzugt sind solche Agglomeratoren, die eine kontinuierliche Fahrweise ermöglichen, insbesondere Wirbelschichtgranulatoren. Die kontinuierliche Aufbaugranulierung in der Wirbelschicht wird in einem mehrstufigen, vorzugsweise 2 bis 5stufigen Granulator bei Verweilzeiten von 20 bis 60 min und Zulufttemperaturen von 60 bis 100 °C, vorzugsweise 65 bis 85 °C, durchgeführt.

Die genaue Einstellung der Zulufttemperatur erfolgt so, daß die Temperatur im Wirbelbett bei der Granulierung zwischen 25 bis 60 °C, vorzugsweise 30 bis 40 °C, liegt. Erfindungsgemäß besonders vorteilhaft ist die Entfernung von Wasser während des Granuliervorganges in der Wirbelschicht. Dadurch lassen sich Granulate mit bis zu 43 % Diperoxidicarbonsäure herstellen, ohne daß es zu Verklebungen und Anbackungen kommt.

Der Wassergehalt der anfallenden feuchten Granulate kann je nach Verfahrensvariante bis zu 35 % betragen. Zur weitgehenden Entfernung der Feuchtigkeit ist erfindungsgemäß die kontinuierliche Trocknung in der Wirbelschicht besonders bevorzugt. Eingesetzt werden mehrstufige, bevorzugt 2 bis 5stufige, und insbesondere 3stufige Wirbelschichttrockner. Die Zulufttemperatur beträgt zu Beginn 90 bis 140 °C und wird entsprechend der gewählten Stufenzahl bis auf 50 bis 90 °C reduziert. Die Einstellung der Zulufttemperatur richtet sich nach der Temperatur im Wirbelbett, die zur gefahrlosen Durchführung höchstens 60 °C, vorzugsweise höchstens 55 °C, betragen darf. Die Verweilszeiten liegen zwischen 10 bis 60 min, wobei Granulate mit Restfeuchten von weniger als 10 %, vorzugsweise weniger als 5 %, anfallen.

Durch diese Maßnahmen erhält man einen Bleichwirkstoff, der mit prozeßeigenem Natriumsulfat phlegmatisiert ist. Der Bleichwirkstoff ist gekennzeichnet durch einen Gehalt von weniger als 50 ppm Chloridionen sowie einen tolerierbaren Gehalt von bis zu 30 ppm Schwermetallen. Als zusätzliche stabilisierende Additive enthält er darüberhinaus, bezogen auf die Diperoxidicarbonsäure 2 bis 200 %, vorzugsweise 5 bis 50 %, eines wasserlöslichen organischen Polymeren, 0 bis 120 %, vorzugsweise 2 bis 20 %, eines Phosphates, vorzugsweise Natriumtripolyphosphat, 0 bis 150 %, vorzugsweise 20 bis 100 %, Magnesiumsulfat, 0 bis 10 %, vorzugsweise 0 bis 3 %, eines Komplexbildners für Schwermetalle und 0 bis 20 %, vorzugsweise 0 bis 5 %, eines Silikates. Diese Bestandteile werden durch das erfindungsgemäße Verfahren nicht homogen über die Granulate verteilt, sondern in räumlicher Nachbarschaft zur Diperoxidicarbonsäure angereichert. Die beschriebenen Maßnahmen bewirken einen ausgeprägten synergistischen Effekt, so daß sich der Bleichwirkstoff durch erstaunliche chemische Stabilität und durch geringe Zersetzungsneigung beim Erwärmen auszeichnet.

Bevorzugtes Einsatzgebiet dieses Bleichwirkstoffes ist die Bleiche von Textilien und Fasern. Hierbei kann der Bleichwirkstoff an sich oder aber in Kombination mit Waschmitteln eingesetzt werden. Besonders vorteilhaft ist die Konfektionierung mit Waschmitteln, zumal der Bleichwirkstoff hinsichtlich Korngrößenverteilung, Schüttgewicht und mechanischer Stabilität der für Waschmittelbestandteile geforderten Spezifikation weitgehend entspricht und darüberhinaus über eine ausgezeichnete chemische Stabilität verfügt.

## Beispiele

Lagerstabilität und Zersetzungsneigung von Diperoxidicarbonsäuren werden durch Chloridionen beeinträchtigt.

Die in den Beispielen eingesetzten Ausgangsmaterialien enthielten die nachfolgend aufgeführten Konzentrationen an Chloridionen:

|  | Chloridionen ppm |
|---|---|
| 96%ige Schwefelsäure | < 1 |
| 40%iges Wasserstoffperoxid | < 5 |
| Dodecandisäure | < 1 |
| Brassylsäure | < 2 |
| 50%ige Natronlauge | 20 |
| 30%ige Natronlauge | 12 |
| Magnesiumsulfat-Heptahydrat | 45 |
| Natriumtripolyphosphat | 160 |
| Polyacrylsäure | < 3 |
| Ethylen-Maleinsäure-Copolymer | < 5 |

Die nachfolgenden Beispiele erläutern das erfindungsgemäße Verfahren.

Beispiel 1

Herstellung einer Reaktionsmischung mit Diperoxidodecandisäure (DPDDS)

In einem Rührkessel wurde eine Mischung aus 5 750 g 96%iger Schwefelsäure und 2 350 g 40%igem Wasserstoffperoxid vorgelegt und bei 45 °C innerhalb von 30 min mit 1 060 g gepulverter Dodecandisäure (DDS) versetzt. Nach 4 h Reaktionszeit wurde eine Mischung folgender Zusammensetzung erhalten:

| 11,5 % | Feststoff | (97,4 % DPDDS + 2,6 % DDS) |
|--------|-----------|----------------------------|
| 88,5 % | Flüssigkeit | (67,2 % $H_2SO_4$, 4,1 % $H_2O_2$, 27,3 % $H_2O$) |

Beispiel 2

Kontinuierliche Filtration der Reaktionsmischung

In einer Abb. 1 entsprechenden Filtrationskaskade wurden kontinuierlich 5 kg/h Reaktionsmischung, bestehend aus 52,0 % $H_2SO_4$, 19,4 % DPDDS, 0,3 % DDS, 1,3 % $H_2O_2$, 0,29 % wasserlösliche organische Bestandteile und 26,0 % $H_2O$ (Feststoffanteil 19,7 %, Zusammensetzung 98,5 % DPDDS, 1,5 % DDS) in den Mischbehälter A sowie 6 kg $H_2O$ in den Mischbehälter E eingespeist. Die Pumpleistung wird auf etwa 7 bis 7,5 l/h eingestellt, so daß in jeder Filtrationsstufe bis auf einen Feststoffgehalt von ca. 25 % eingedickt wird. Das Filtrat der Filtrationseinheiten F bzw. D wird vollständig in die Mischbehälter C bzw. A zurückgeführt. Nach Erreichen des stationären Zustands verläßt eine 35,1%ige $H_2SO_4$ (7 kg/h) als Filtrat die Filtrationsstufe B sowie 4 kg/h einer Suspension mit 25,2 % Feststoffanteil und einem Rest-$H_2SO_4$-Gehalt von 4,9 % (bezogen auf die flüssigen Anteile) die Filtrationsstufe F. Die Suspension wurde im nachgeschalteten Rührkessel G durch Zugabe von 315 g/h 50%iger NaOH kontinuierlich auf pH 3,5 neutralisiert, so daß eine Suspension folgender Zusammensetzung erhalten wurde.

| 22,9 % | DPDDS |
|--------|-------|
| 0,5 % | DDS |
| 5,3 % | $Na_2SO_4$ |
| 71,1 % | $H_2O$ |

wasserunlöslicher Feststoffanteil 23,4 % aus 97,9 % DPDDS und 2,1 % DDS
Filtrat: 35,1%ige $H_2SO_4$

Beispiel 3

Neutralisation des Filtrates und Kristallisation

In einem Rührkessel wurden 200 kg der gemäß Beispiel 2 als Filtrat anfallenden 35,1%igen $H_2SO_4$ bei 40 °C durch Zugabe von 115 kg 50%iger Natronlauge innerhalb von 4 h auf pH 3,5 neutralisiert. Man erniedrigte die Temperatur innerhalb von 8 h auf ca. 3 °C, zentrifugierte und erhielt so 214 kg eines feuchten Glaubersalzes. Das Glaubersalz wurde bei 50 °C aufgeschmolzen und die erzeugte Schmelze vom Bodensatz abdekantiert. Bei 80 °C führte man mit der Schmelze im Vakuum eine Verdampfungskristallisation durch, bis ein Feststoffgehalt von etwa 47 % erreicht wurde. Nach Zentrifugieren und Trocknen erhielt man 62 kg $Na_2SO_4$ mit einem Restfeuchtegehalt von 0,8 %, einem Schüttgewicht von 1 230 g/l und eine Korngrößenverteilung von 90 % unterhalb 100 μm. Der Gehalt an Schwermetallen betrug 20 ppm und der Chloridgehalt 5 ppm.

Die Nachteile der direkten Neutralisation des Reaktionsaustrages werden anhand der Beispiele 4 und 5 (Vergleichsbeispiele) verdeutlicht.

Beispiel 4(Vergleichsbeispiel

In situ Phlegmatisierung von DPDDS durch direkte Neutralisation der Reaktionsmischung

4 500 g der nach Beispiel 1 anfallenden Reaktionsmischung verdünnte man mit 5 250 g Wasser und neutralisierte innerhalb von 3 h bei 40 °C mit 30%iger NaOH auf pH 3,5. Nach Kühlung auf 20 °C wurde zentrifugiert und bei 45 °C getrocknet. Man erhielt 1 650 g mit $Na_2SO_4$ phlegmatisierte DPDDS.

| Zusammensetzung: | 25,9 % | DPDDS |
|---|---|---|
| | 2,6 % | DDS |
| | 67,4 % | $Na_2SO_4$ |
| | 3,7 % | $H_2O$ |

Bilanz der wasserlöslichen Bestandteile

| Zusammensetzung vor der Neutralisation: | 97,4 % | DPDDS, | 2,6 % | DDS |
|---|---|---|---|---|
| Zusammensetzung nach der Neutralisation: | 90,0 % | DPDDS, | 9,1 % | DDS |

Weitere Analysendaten enthalten die Tabellen 1, 2 und 3.

Beispiel 5 (Vergleichsbeispiel)

4 500 g der nach Beispiel 1 anfallenden Reaktionsmischung versetzte man mit 5 250 g 30%iger $Na_2SO_4$-Lösung und neutralisierte innerhalb von 3 h bei 40 °C mit 30%iger NaOH auf pH 3,5. Nach Kühlung auf 20 °C wurde zentrifugiert und bei 45 °C getrocknet. Man erhielt 3 182 g mit $Na_2SO_4$ phlegmatisierte DPDDS.

| Zusammensetzung: | 13,1 % | DPDDS |
|---|---|---|
| | 2,2 % | DDS |
| | 80,2 % | $Na_2SO_4$ |
| | 4,2 % | $H_2O$ |

Bilanz der wasserlöslichen Bestandteile:

| Zusammensetzung vor der Neutralisation: | 97,4 % | DPDDS, | 2,6 % | DDS |
|---|---|---|---|---|
| Zusammensetzung nach der Neutralisation: | 85,6 % | DPDDS, | 14,4 % | DDS |

Weitere Analysendaten enthalten die Tabellen 1, 2 und 3.

Beispiel 6

Aufbaugranulierung mit Diperoxidodecandisäure

Einen 15 l-Patterson-Kelley V-Mischer beschickte man mit 6 kg des nach Beispiel 3 anfallenden Natriumsulfates. Bei 50 UpM wurden innerhalb von 13 min 1 800 g einer auf pH 4 eingestellten DPDDS-Suspension, bestehend aus 32,0 % DPDDS, 0,3 % DDS, 3,9 % $Na_2SO_4$, 4,8 % Ethylen-Maleinsäure-Copolymer (Mw ca. $10^4$), 3,2 % $Na_5P_3O_{10}$ und 56,1 % $H_2O$ eingedüst. Man granulierte noch 3 min bei 30 UpM nach und trocknete 20 min in der Wirbelschicht. Der Zuluftstrom betrug 80 m³/h. Die Temperatur der Zuluft lag zu Trocknungsbeginn bei 120 °C und wurde im Verlauf der Trocknung auf ca. 70 °C erniedrigt, so daß die Wirbelschicht Temperaturen zwischen 35 bis 40 °C aufwies.

| Zusammensetzung der Granulate: | 8,5 % | DPDDS |
|---|---|---|
| | 0,1 % | DDS |
| | 1,3 % | Ethylen-Maleinsäure-Copolymer |
| | 0,8 % | $Na_5P_3O_{10}$ |
| | 86,0 % | $Na_2SO_4$ |
| | 3,2 % | $H_2O$ |

Bilanz der wasserunlöslichen Bestandteile:

| Verhältnis DPDDS/DDS vor der Granulierung: | 99,1 | : | 0,9 |
|---|---|---|---|
| Verhältnis DPDDS/DDS nach der Granulierung: | 98,8 | : | 1,2 |

Weitere Analysendaten enthalten die Tabellen 1, 2 und 3.

Beispiel 7

Aufbaugranulierung mit Diperoxidodecandisäure

In ein Wirbelbett aus 5 kg des nach Beispiel 3 hergestellten $Na_2SO_4$ wurden 5 kg der nach Beispiel 2 erhaltenen DPDDS-Suspension (22,9 % DPDDS, 0,5 % DDS, 5,3 % $Na_2SO_4$, 71,1 % $H_2O$) innerhalb von 20 min eingedüst. Der Zulaufstrom lag zu Beginn der Granulierung bei ca. 50 m³/h erhöht. Die Zulufttemperatur betrug ca. 75 °C, so daß die Temperatur im Wirbelbett zwischen 30 bis 35 °C lag. Nach der Granulierung wurde 20 min bei einem Zuluftstrom von ca. 80 m³/h und einer Zulufttemperatur von ca. 80 °C nachgetrocknet, wobei sich die Temperatur der Wirbelschicht auf ca. 50 °C erhöhte.

| Zusammensetzung der Granulate: | 18,2 % | DPDDS |
|---|---|---|
| | 0,4 % | DDS |
| | 79,0 % | $Na_2SO_4$ |
| | 2,4 % | $H_2O$ |

Bilanz der wasserunlöslichen Bestandteile:

| Verhältnis DPDDS/DDS vor der Granulierung: | 97,9 | : | 2,1 |
|---|---|---|---|
| Verhältnis DPDDS/DDS nach der Granulierung: | 97,8 | : | 2,2 |

Weitere Analysendaten enthalten die Tabellen 1, 2 und 3.

Beispiele 8 bis 10

Beispiele für Granulate mit Diperoxidodecandisäure

Analog zu Beispiel 7 wurden in der Wirbelschicht verschiedene DPDDS/$Na_2SO_4$-Granulate hergestellt, die sich zum einen durch die Qualität des eingesetzten $Na_2SO_4$ und zum anderen durch den Zusatz von Additiven (wie $MgSO_4$, $Na_5P_3O_{10}$, wasserlöslichen Polymeren) zur DPDDS-Suspension vor Durchführung der Granulierung voneinander unterscheiden. Diese Maßnahmen führen zwar zu keiner signifikanten Veränderung des Granulier- und Trocknungsprozesses, jedoch wird eine deutliche Beeinflussung der Granulateigenschaften ersichtlich, die in den Tabellen 1, 2 und 3 erläutert werden.

Beispiel 11

Aufbaugranulat mit Diperoxibrassylsäure

Die Veruchsdurchführung entspricht der im Beispiel 7 angeführten Verfahrensweise. Eingesetzt wurden 5 kg $Na_2SO_4$, auf das 6 kg Diperoxibrassylsäuresuspension, bestehend aus 21,4 % Diperoxibrassylsäure, 0,8 % Brassylsäure, 5,2 % $Na_2SO_4$, 3,2 % Polyacrylsäure (Mw $10^5$); 1,0 % $Na_5P_3O_{10}$, 5,1 % $MgSO_4$ und 63,3 % $H_2O$, innerhalb von 20 min aufgesprüht wurden. Die Zusammensetzung der Granulate und weitere Analysendaten enthalten die Tabellen 1, 2 und 3.

Tabelle 1

| Zusammensetzung der Persäuregranulate in Gewichtsprozent | | | | | | | | |
|---|---|---|---|---|---|---|---|---|
| Beispiel | 4 | 5 | 6 | 7 | 8 | 9 | 10 | 11 |
| Persäure (potentiometrisch) | 25,9 | 13,1 | 8,5 | 18,2 | 18,4 | 17,8 | 23,1 | 17,9 |
| Ausgangscarbonsäure | 2,6 | 2,2 | 0,1 | 0,4 | 0,4 | 0,4 | 0,5 | 0,7 |
| $Na_2SO_4$ | 67,4 | 80,2 | 86,0 | 79,0 | 75,9 | 74,2 | 62,3 | 70,1 |
| $MgSO_4$ | - | - | - | - | - | 4,3 | 5,6 | 4,3 |
| $Na_5P_3O_{10}$ | - | - | 0,8 | - | - | 0,8 | 1,1 | 0,8 |
| Polyacrylsäure | - | - | 1,3 | - | 2,7 | - | 3,5 | - |
| Ethylen-Maleinsäure-Copolymer | - | - | - | - | - | 2,7 | - | 2,7 |
| $H_2O$ | 3,7 | 4,2 | 3,2 | 2,4 | 2,6 | 3,8 | 3,9 | 3,4 |
| Schwermetallgehalt ppm | 23 | 24 | 20 | 20 | 21 | 20 | 20 | 19 |
| Chloridgehalt ppm | < 5 | < 5 | < 1 | < 1 | < 1 | < 1 | < 1 | < 1 |
| Fraktion 0,2 - 1,0 mm (%) | 87,5 | 89,1 | 94,2 | 62,8 | 90,5 | 96,4 | 97,0 | 91,2 |
| Schüttgewicht (g/l) der Fraktion 0,2 - 1,0 mm | 990 . | 1 030 | 575 | 915 | 535 | 495 | 580 | 615 |
| Abrieb < 200 mm nach 2 h (%) | - | - | 2,9 | 65,0 | 2,1 | 1,4 | 1,9 | 5,2 |

Tabelle 2: Lagerstabilität der Persäuregranulate

Zur Bestimmung der Lagerstabilität wurden je 200 g Persäuregranulat bei 30 bzw. 50 °C gelagert. Zu Beginn, sowie nach 10, 30 und 60 Tagen Lagerzeit wurde die Persäure potentiometrisch durch Titration mit NaOH bestimmt. Bei Anwesenheit von Magnesiumsulfat wurden die Granulate in Isopropanol/Wasser (80 : 20) aufgerührt und filtriert. Im Filtrat bestimmt man dann potentiometrisch den Persäuregehalt. Die Versuchsergebnisse zeigen, daß die Verwendung des prozeßeigenen $Na_2SO_4$ zu deutlicher Verbesserung der Lagerstabilität führt. Bei zusätzlicher Abschirmung der Persäure durch inerte wasserlösliche Polymere und/oder Zusätze von $MgSO_4$ und/oder $Na_5P_3O_{10}$ werden Granulate erhalten, die sich selbst bei höherer Temperatur durch hervorragende Lagerstabilität auszeichnen.

| Lagerstabilität bei 50 °C (Persäuregehalt in % bezogen auf den Anfangsgehalt) | | | | | | | | | |
|---|---|---|---|---|---|---|---|---|---|
| Beispiel | 4 | 5 | 6 | 7 | 8 | 9 | 10 | 11 | 12 |
| Lagerzeit (Tage) | | | | | | | | | |
| 10 | 4,0 | 3,9 | 87 | 71 | 5,8 | 88 | 90,0 | 91,5 | 88,5 |
| 30 | - | - | 77 | 43 | | 78 | 82 | 85 | 81 |
| 60 | - | - | 69 | - | | 67 | 76 | 79 | 70 |

| Lagerstabilität bei 30 °C | | | | | | |
|---|---|---|---|---|---|---|
| Beispiel | 6 | 7 | 9 | 10 | 11 | 12 |
| Lagerzeit (Tage) | | | | | | |
| 10 | 99 | 94 | 97 | 98 | 99 | 96 |
| 30 | 95,5 | 85 | 92 | 96 | 98,5 | 94 |
| 60 | 94 | 76 | 87 | 92 | 95,5 | 91,5 |

Tabelle 3: Bestimmung der Zersetzungstemperatur

Zur Bestimmung wurden die Granulate 24 h im Vakuum bei Raumtemperatur getrocknet. Jeweils 10 g Granulat füllte man in einen Aluminiumblock und heizte mit 2,5 °C/min auf. Gemessen wurde die Temperatur, bei der sich die Probe unter exothermer Reaktion zersetzte. Die Versuche zeigen, daß sich durch Verwendung eines prozeßeigenen $Na_2SO_4$ nach dem erfindungsgemäßen Verfahren eine deutliche Erhöhung der Zersetzungstemperatur erzielen läßt, die insbesondere durch Zusatz von bestimmten Polycarboxylaten sowie $MgSO_4$ und $Na_5P_3O_{10}$ noch weiter gesteigert werden kann.

| Zersetzungstemperatur von Persäuregranulaten | | | | | | | | |
|---|---|---|---|---|---|---|---|---|
| Beispiel | 4 | 5 | 6 | 7 | 8 | 9 | 10 | 11 |
| Temperatur (°C) | 92 | 95 | 123 | 107 | 135 | 125 | 130 | 129 |

**Ansprüche**

1. Verfahren zur Herstellung eines wasserunlösliche Diperoxidicarbonsäure enthaltenden phlegmatisierten Bleichmittels,
dadurch gekennzeichnet,
daß man

a) den Reaktionsaustrag der mittels Wasserstoffperoxid/Schwefelsäure durchgeführten Peroxigenierung von wasserunlöslichen Dicarbonsäuren, mit oder ohne Verdünnen mit Wasser, durch kontinuierliche Filtration in einer mehrstufigen Filtrationskaskade, die das Auftreten von Zonen mit nahezu trockener Diperoxidicarbonsäure ausschließt, auftrennt in ein die Hauptmenge an Schwefelsäure enthaltendes, weitgehend peroxidfreies Filtrat und eine bezogen auf die flüssige Phase der Suspension weniger als 10 % Schwefelsäure enthaltende Diperoxidicarbonsäuresuspension,

b) aus der als Filtrat erhaltenen Schwefelsäure in an sich bekannter Weise nach Neutralisation auf pH 2 bis 6 mit bis zu 50%iger wäßriger Natronlauge, die bis zu 30 ppm Schwermetalle und weniger als 50 ppm Chloridionen enthält, eine Natriumsulfatlösung herstellt, aus der, mit oder ohne Verdünnen mit Wasser oder Aufkonzentrieren, durch Kühlungskristallisation zunächst Glaubersalz und anschließend durch Verdampfungskristallisation ein Natriumsulfat mit einem Schüttgewicht von 1 000 bis 1 500 g/l und einer Korngrößenverteilung von mindestens 90 % unterhalb 200 $\mu$m gewonnen wird, das bis zu 30 ppm Schwermetalle, bis zu 50 ppm Chloridionen und weniger als 5 % Wasser enthält,

c) die Schwefelsäure der nach Filtration anfallenden Diperoxidicarbonsäuresuspension bei Temperaturen unterhalb von 40 °C, durch Zugabe bis zu 50%iger wäßrige Natronlauge, die bis zu 30 ppm Schwermetalle und bis zu 50 ppm Chloridionen enthält, auf pH 2 bis 6 neutralisiert,

d) der nach Neutralisation erhaltenen Diperoxidicarbonsäuresuspension, bezogen auf den Gehalt an Diperoxidicarbonsäure, 2 bis 200 % eines wasserlöslichen organischen Polymeren zusetzt, wobei die Schwermetallkonzentration in der Diperoxidicarbonsäuresuspension nach Zugabe des Polymeren bis zu 30 ppm und die Chloridionenkonzentration bis zu 50 ppm beträgt,

e) und die Diperoxidicarbonsäure mit einer Schicht, bestehend aus dem unter d) zugesetzten Polymeren umgibt, wobei man die gemäß d) erzeugte wäßrige Diperoxidicarbonsäuresuspension durch Vermischen mit dem unter b) hergestellten Natriumsulfat in geeigneten Apparaturen dehydratisiert, so daß gleichzeitig Aufbaugranulate entstehen, die nach dem schonenden Trocknen zu mindestens 90 % Korngrößen im Bereich von 200 bis 1 250 $\mu$m und Schüttgewichte zwischen 400 bis 800 g/l aufweisen und die bei zweistündiger Messung der Abriebfestigkeit auf einem Engelsmann-Taumelsieb der Maschenweite 200 $\mu$m weniger als 6 % Abrieb liefern.

2. Verfahren nach Anspruch 1,
dadurch gekennzeichnet,
daß als organische Diperoxidicarbonsäuren alpha,omega-Diperoxidicarbonsäuren mit 9 bis 13 Kohlenstoffatomen oder Gemische davon verwendet werden.

3. Verfahren nach Anspruch 2,
dadurch gekennzeichnet,
daß als organische Diperoxidicarbonsäuren Diperoxidodecandisäure und/oder Diperoxibrassylsäure verwendet werden.

4. Verfahren nach Anspruch 1 a,
dadurch gekennzeichnet,
daß man den Reaktionsaustrag herstellt durch kontinuierliche Peroxigenierung in einer mehrstufigen Rührkesselkaskade, indem man die wasserunlösliche Dicarbonsäure, vorzugsweise in fester Form, in eine Mischung, bestehend aus 50 bis 70 % Schwefelsäure, 4 bis 20 % Wasserstoffperoxid und 10 bis 46 % Wasser einspeist, wobei ein insgesamt über die Ausgangsmaterialien in die Reaktionsmischung eingebrachter Schwermetallgehalt von bis zu 40 ppm und ein Chloridgehalt von bis zu 20 ppm auftritt worauf man die Reaktion bei Temperaturen von 30 bis 50 °C und Verweilzeiten von 4 bis 14 Stunden durchführt und die in der Reaktionsmischung gelösten Verunreinigungen durch oxidativen Abbau der Säure unter 1 % liegen.

5. Verfahren nach Anspruch 4,
dadurch gekennzeichnet,
daß man den Reaktionsaustrag durch kontinuierliche Peroxigenierung in einer dreistufigen Rührkesselkaskade herstellt.

6. Verfahren nach Anspruch 1 a,
dadurch gekennzeichnet,
daß man den Reaktionsaustrag, im wesentlichen bestehend aus 1 bis 25 % Diperoxidicarbonsäure und einer 50 bis 70%igen Schwefelsäure als flüssiger Phase sowie Resten an überschüssigem Wasserstoffperoxid und nicht umgesetzter Ausgangscarbonsäure mit 0,1 bis 5,5 Teilen Wasser pro Teil Reaktionsaustrag verdünnt und anschließend durch mehrstufige, kontinuierliche Filtration unter Filtratrückführung in die jeweils vorhergehende Filtrationsstufe unter Vermeidung von Zonen mit nahezu trockener Diperoxidicarbonsäure auftrennt in eine bezogen auf die flüssigen Anteile weniger als 10 % Schwefelsäure enthaltende Suspension mit einem auf die Gesamtsuspension bezogenen Gehalt an Diperoxidicarbonsäure von 10 bis 40 %, und ein 10 bis 40 % Schwefelsäure enthaltendes, nahezu peroxidfreies Filtrat.

7. Verfahren nach Anspruch 6,

dadurch gekennzeichnet,

daß man den Reaktionsaustrag mit 0,5 bis 2,5 Teilen Wasser verdünnt.

8. Verfahren nach Anspruch 6,

dadurch gekennzeichnet,

daß man den Reaktionsaustrag durch 2- bis 4stufige kontinuierliche Filtration auftrennt.

9. Verfahren nach Anspruch 6,

dadurch gekennzeichnet,

daß man den Reaktionsaustrag durch 3stufige kontinuierliche Filtration auftrennt.

10. Verfahren nach Anspruch 6,

dadurch gekennzeichnet,

daß die Diperoxidicarbonsäuresuspension weniger als 5 % Schwefelsäure bezogen auf den Gehalt an Diperoxidicarbonsäure enthält.

11. Verfahren nach Anspruch 6,

dadurch gekennzeichnet,

daß der Gehalt an Diperoxidicarbonsäure der Diperoxidicarbonsäuresuspension 20 bis 35 % beträgt.

12. Verfahren nach Anspruch 6,

dadurch gekennzeichnet,

daß das nahezu peroxidfreie Filtrat 25 bis 35 % Schwefelsäure enthält.

13. Verfahren nach Anspruch 6,

dadurch gekennzeichnet,

daß man die Filtration mit bewegten Filterkerzen ausführt und damit gleichzeitig die zu filtrierende Suspension in ausreichendem Maße rührt oder nach dem Prinzip der Querstrommikrofiltration ausführt.

14. Verfahren nach Anspruch 13,

dadurch gekennzeichnet,

daß die bewegten Filterkerzen radial angeordnet sind.

15. Verfahren nach Anspruch 1 b,

dadurch gekennzeichnet,

daß das Natriumsulfat eine Korngrößenverteilung von mindestens 90 % unterhalb 100 $\mu$m aufweist.

16. Verfahren nach Anspruch 1 c,

dadurch gekennzeichnet,

daß man die Schwefelsäure bei Temperaturen unterhalb 20 °C neutralisiert.

17. Verfahren nach Anspruch 1 c,

dadurch gekennzeichnet,

daß man bei der Neutralisation der nach Filtration erhaltenen Diperoxidicarbonsäuresuspension die Natronlauge teilweise oder vollständig durch Magnesiumoxid oder Magnesiumhydroxid ersetzt.

18. Verfahren nach Anspruch 1 d,

dadurch gekennzeichnet,

daß man 5 bis 50 % eines wasserlöslichen Polymeren zusetzt.

19. Verfahren nach Anspruch 1 d,

dadurch gekennzeichnet

daß das wasserlösliche Polymere eine polymere Carbonsäure ist.

20. Verfahren nach Anspruch 19,

dadurch gekennzeichnet,

daß das wasserlösliche Polymer ein Homopolymerisat und/oder Copolymerisat ungesättigter Carbonsäuren ist.

21. Verfahren nach Anspruch 20,

dadurch gekennzeichnet,

daß die ungesättigte Carbonsäuren ausgewählt sind aus Acrylsäure, Maleinsäure und Crotonsäure.

22. Verfahren nach Anspruch 20,

dadurch gekennzeichnet,

daß die Copolymerisate aus den ungesättigten Carbonsäuren mit Ethylen, Propylen, Buten, Vinylacetat und/oder Styrol bestehen.

23. Verfahren nach Anspruch 1 d,

dadurch gekennzeichnet,

daß man der nach Neutralisation erhaltenen Diperoxidicarbonsäuresuspension bekannte Additive zusetzt, wobei die Schwermetallkon zentration in der Diperoxidicarbonsäuresuspension nach Zugabe der Additive bis zu 30 ppm sowie Chloridionenkonzentration bis zu 50 ppm beträgt.

24. Verfahren nach Anspruch 1 e,
dadurch gekennzeichnet
daß die Additive in räumlicher Nachbarschaft zur Diperoxidicarbonsäure angeordnet sind.

25. Verfahren nach Anspruch 1 e,
dadurch gekennzeichnet,
daß der Gehalt an Diperoxidicarbonsäure in den getrockneten Granulaten im Bereich von 5 bis 43 % liegt.

26. Verfahren nach Anspruch 24,
dadurch gekennzeichnet,
daß der Gehalt an Diperoxidicarbonsäure 12 bis 30 % beträgt.

27. Verfahren nach Anspruch 1 e,
dadurch gekennzeichnet,
daß man die Dehydratisierung/Granulierung kontinuierlich in einem mehrstufigen Wirbelschichtgranulator bei Verweilzeiten von 20 bis 60 min, Zulufttemperaturen von 60 bis 100 °C, und Temperaturen im Wirbelbett von 25 bis 60 °C durchführt, durch Einspeisen einer Diperoxidicarbonsäure enthaltende wäßrige Suspension in ein Natriumsulfat-Wirbelbett.

28. Verfahren nach Anspruch 26,
dadurch gekennzeichnet,
da man einen 2- bis 5stufigen Wirbelschichtgranulator verwendet.

29. Verfahren nach Anspruch 26,
dadurch gekennzeichnet,
daß die Zulufttemperatur 65 bis 80 °C beträgt.

30. Verfahren nach Anspruch 26,
dadurch gekennzeichnet,
daß die Wirbelbettemperatur 30 bis 40 °C beträgt.

31. Verfahren nach Anspruch 1 e,
dadurch gekennzeichnet,
daß man die Dehydratisierung/Granulierung nach einem Sprühmischverfahren durchführt, durch Beaufschlagung bewegten Natriumsulfates mit einer Diperoxidicarbonsäure enthaltenden wäßrigen Suspension.

32. Verfahren nach Anspruch 1 e,
dadurch gekennzeichnet,
daß man die Trocknung der diperoxidicarbonsäurehaltigen Granulate kontinuierlich in einem mehrstufigen Wirbelschichttrockner bei Verweilzeiten von 10 bis 60 min durchführt, wobei die Zulufttemperatur von 90 bis 140 °C in der 1. Stufe stufenweise entsprechend der gewählten Stufenzahl auf 50 bis 90 °C reduziert wird, so daß die Temperatur im Wirbelbett 60 °C nicht übersteigt, und man dabei Granulate mit einer Restfeuchte von weniger als 10 % erhält.

33. Verfahren nach Anspruch 31,
dadurch gekennzeichnet,
daß man Granulate mit einer Restfeuchte von weniger als 5 % erhält.

34. Verwendung eines wasserunlösliche Diperoxidicarobnsäure enthaltenden phlegmatisierten Bleichmittels nach den Ansprüchen 1 bis 33 zur Bleiche von Textilien und Fasern.

35. Verwendung nach Anspruch 34 in Kombination mit Waschmitteln.

Abbildung 1

Abbildung 2

EP 0 375 829 A2